Europäisches Patentamt

European Patent Office

Office européen des brevets

(19)

(11) Veröffentlichungsnummer: **0 295 331 B1**

# (12) EUROPÄISCHE PATENTSCHRIFT

(45) Veröffentlichungstag der Patentschrift: **29.04.92**

(21) Anmeldenummer: **87116457.0**

(22) Anmeldetag: **07.11.87**

(51) Int. Cl.⁵: **A61K 31/355**, //(A61K31/355, 31:23,31:20)

(54) **Verwendung von Vitamin E zur Herstellung von Arzneimitteln zur Normalisierung der Blutgerinnung bei Therapie mit hochungesättigten Fettsäuren vom Omega-3-Typ.**

(30) Priorität: **06.06.87 DE 3719097**

(43) Veröffentlichungstag der Anmeldung:
**21.12.88 Patentblatt 88/51**

(45) Bekanntmachung des Hinweises auf die Patenterteilung:
**29.04.92 Patentblatt 92/18**

(84) Benannte Vertragsstaaten:
**AT BE CH DE ES FR GB GR IT LI LU NL SE**

(56) Entgegenhaltungen:
**DE-A- 3 213 744**
**FR-A- 2 231 379**

**PATENT ABSTRACTS OF JAPAN, Band 9, Nr. 278 (C-312)[2001], 6. November 1985; & JP-A-60 123 414 (MIDORI JUJI K.K.) 02-07-1985**

**DIALOG 76206985, K. BENGTSEN et al.: "Effects of dietary alpha tocopherol and polyunsaturated fats on the fatty acid composition of platelet phospholipids and on blood coagulation", & THROMB. DIATHES. HEAMORRH (GERMANY, WEST),1975, 34/3**

(897-898)

(73) Patentinhaber: **Fratzer, Uwe, Dr. med.
Lauberweg 29
W-6719 Hettenleidelheim(DE)**

(72) Erfinder: **Fratzer, Uwe, Dr. med.
Lauberweg 29
W-6719 Hettenleidelheim(DE)**

(74) Vertreter: **Grussdorf, Jürgen, Dr. et al
Patentanwälte Zellentin & Partner Rubensstrasse 30
W-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung ist die Verwendung von Vitamin E zur Herstellung von Arzneimitteln zur Normalisierung der Blutgerinnung bei Therapie mit hochungesättigten Fettsäuren vom Omega-3-Typ.

Ausgelöst durch Untersuchungen über das Ernährungsverhalten von Grönland-Eskimos, wurde in den 60 und 70iger Jahren ein Zusammenhang zwischen dem Konsum von Fischöl und der Verringerung von Myocard- und Hirninfarkten beobachtet Als essentielle Bestandteile der Fischöle erwiesen sich die darin enthaltenen hochungesättigten Fettsäuren vom Omega-3-Typ, insbesondere Eicosapentaensäure (EPA) und Docosahexaensäure (DHA). Weitere Untersuchungen haben ergeben, daß diese Stoffe thrombozytenaggregationshemmend wirken, indem sie anstelle von Arachidonsäure (Eicosatetraensäure, AA) in die Thrombozytenmembran eingebaut werden, wodurch sich die rheologischen Eigenschaften des Blutes Positiv verändern Darüber hinaus wurde eine Senkung von Blutzuckerwerten, die Bildung entzündungshemmender Leukotriene und eine Senkung erhöhter Blutdruckwerte beobachtet Ein für das Infarktrisiko wichtiger Befund ist, daB unter Dauerapplikation solcher hochungesättigten Fettsäuren in Mengen von etwa 50-1000 mg/Tag Blutcholesterinwerte bis 35 % und Triglyceride bis 58 % gesenkt werden. Bei der Cholesterinsenkung werden insbesondere die "low density lipoprotein (LDL)" vermindert, während die "high density lipoprotein (HDL)", die dem Infarktrisiko entgegenwirken, relativ erhöht werden.

Als Nebenwirkung wurde beobachtet, daß Eskimos aufgrund der Fischdiät eine erhöhte Blutungstendenz aufweisen (R. Saynor und D. Verell, IRCS, Medical Science 8, 379-379 (1980). In ihren Untersuchungen finden die Autoren jedoch weder eine signifikante Veränderung der partiellen Thromboplastinzeit noch der Thrombingerinnungszeit. Dagegen wird von T. Terano et al. Atherosclerosis, 46 (1983), 321-331 eine signifikante Erhöhung der Prothrombinzeit von 11,5 auf 12,6 Sek. gefunden, während die partielle Prothrombinzeit nicht verändert ist. Diese Autoren finden darüber hinaus keine signifikante Änderung bei Cholesterin, Phospholipiden, HDL-Cholesterin, Malondialdehyd und Vitamin E-Gehalt im Serum. Durch eigene Untersuchungen an einem Kollektiv von über 100 Patienten konnte bestätigt werden, daß unter einer Therapie mit hochungesättigten Fettsäuren über einen Zeitraum von 4 oder mehr Wochen die Prothrombinzeit gemessen als "Quickwert" bis zu 45 % unter den Ausgangswert des unbehandelten Patienten absinkt, wenn EPA und/ oder DHA in Mengen bis zu 1000 mg/Tag appliziert werden.

Bei Patienten, die mit Antikoagulantien, wie Indandionen oder Dicumarolen behandelt werden, wird diese Wirkung additiv verstärkt, was zu einem raschen Absinken der Quickwerte unter die therapeutische Sicherheitsgrenze führen und damit zu einem Absetzen der Antikoagulantien zwingen kann.

Da Omega-3-Fettsäuren nicht nur unter ärztlicher Kontrolle bei akuten Erkrankungen, sondern überwiegend als diätetische Mittel vertrieben und von Laien vorbeugend angewendet werden, ist das vorstehende Risiko bedeutend.

Es stellte sich daher die Aufgabe, ein Arzneimittel zu finden, durch welches trotz einer therapeutisch bedingten hohen Applikation von EPA und DHA die Blutgerinnung, gemessen an der Prothrombinzeit, normal bleibt bzw. wieder normalisiert werden kann.

Diese Aufgabe wird durch die in den Ansprüchen näher gekennzeichneten Merkmale gelöst.

Überraschenderweise wurde gefunden, daß Vitamin E (Alpha-Tocopherolacetat) in einer Menge von 40-100 Gew.-% der verabreichten hochungesättigten Fettsäuren ein Absinken des Prothrombinwertes wirksam verhindert bzw. einen bereits abgesunkenen Prothrombinwert in kurzer Zeit wieder normalisiert.

Da hochungesättigte Fettsäuren, wie EPA und DHA relativ oxidationsempfindlich sind, wurde diesen Substanzen bereits früher in einer Menge von 1-2 % ein Antioxidans, insbesondere Vitamin C (Ascorbinsäure) oder Vitamin E zugesetzt. Diese Mengen haben im Gegensatz zu der nunmehr vorgeschlagenen Dosierung keinerlei Einfluß auf die Blutgerinnungszeit.

So sind beispielsweise im Patent Abstracts of Japan, Bd. 9, Nr. 278, S. 2001 bzw. der dort returierten JP-A-60123414 Arzneimittel auf der Basis von EPA beschrieben zur Vorbeugung und Behandlung von Arteriosklerose und Thrombose. Diese Mittel sollen darüber hinaus noch Vitamin E als Antioxidans enthalten, wobei 0,1 - 1 % Vitamin E als ausreichend angesehen wird.

Offensichtlich völlig spekulativ wird noch angegeben, daß das für die Behandlung von Arteriosklerose und Thrombose geeignete Mittel auch 0,01 - 30 % Vitamin E enthalten kann und erwartet wird, daß dieses die gleiche Arzneimittelwirkung wie EPA, d.h. eine antisklerotische und thrombozytenaggregtionshemmende Wirkung besitzt. Daß erfindungsgemäß durch bestimmte Verhältnisse zwischen Vitamin E und ungesättigten Fettsäuren praktisch der gegenteilige Effekt eintritt, d.h. die Störung der Blutgerinnung, welche als Nenbenwirkung der ungesättigten Fettsäure eintritt, aufgehoben wird, läßt sich aus dieser Literaturstelle nicht entnehmen.

Auch die DE-A-3213744 beschreibt Arzneimittel zur Behandlung von Arteriosklerose und Thrombozytenagglutinationshemmung. Auf Seite 8, letzter Absatz wird darauf verwiesen, daß DHA leicht oxidiert und Tocopherol (Vitamin E) als Antioxidans zugesetzt werden kann. Die zuzusetzenden Mengen entsprechen dabei der als Antioxidans üblichen Konzentration im Bereich von 1- 10 %.

Der Reaktionsmechanismus der so in Zusammenhang mit der Gabe von hochungesättigten Fettsäuren verabreichten hohen Dosierungen von Vitamin E ist bisher nicht geklärt worden. Die spontane Quickwerterniedrigung muß mit einer verminderten Prothrombinbildung durch teilweise Bindung des Vitamin K an die hochungesättigten Fettsäuren erklärt werden, welche bei gleichzeitiger Gabe von Vitamin E aufgehoben wird. Dies erklärt auch, warum eine der Menge der ungesättigten Fettsäuren entsprechende Menge Vitamin E appliziert werden muß. Diese Erklärung steht allerdings in Gegensatz zu dem Befund von Saynor et al s.o.), welche bei Applikation von 10 g Fischöl mit einem hohen Gehalt an EPA pro Tag und Versuchsperson auch nach 5 Wochen keine signifikante Veränderung der Prothrombinverhältnisse fanden.

Die Wirkung ist darüber hinaus überraschend, da nicht Vitamin E, sondern Vitamin K das für die Blutgerinnung essentielle Vitamin ist und nach den obigen Befunden auch bei längerer Applikation von EPA und DHA das Vitmin E im Serum nicht geändert wird.

Hochungesättigte Fettsäuren vom Omega-3-Typ (EPA und DHA) werden entweder als Fischölpräparate oder als Konzentrate als Arzneimittel bzw. Diätetika eingesetzt. 5-50 g Fischöl bzw. 50-1000 mg der aus dem Fischöl isolierten oder synthetisch hergestellten Omega-3-Fettsäuren werden üblicherweise täglich verabfolgt. Die entsprechend notwendigen Dosierungen von Vitamin E können entweder in Form der heute bereits üblichen 30-1000 mg Vitamin E (Tocopherolacetat) enthaltenden Präparate parallel eingenommen werden oder, was aus therapeutischen Gründen vorzuziehen ist, dem Fettsäurepräparat direkt zugemischt werden. Wie bereits oben ausgeführt, ist eine Zumischung von 40-100 Gew.-% Vitamin E bezogen auf die essentiellen Omega-3-Fettsäuren notwendig. Größere Mengen Vitamin E haben keine zusätzliche Wirkung und werden daher aus wirtschaftlichen Gründen und um die zu applizierende Dosiseinheit nicht unnötig zu vergrößern, nicht für sinnvoll gehalten. Geringere Mengen, insbesondere die als Antioxidatien gebräuchlichen 1-3 i.U, besitzen keine Wirkung.

Die Applikation erfolgt üblicherweise in Form von Weichgelatinekapseln, Tabletten, Dragees oder in Steckkapseln, wobei selbstverständlich auch ein direktes Auflösen im Fischöl möglich ist. Die Präparationen können zusätzlich die für die Herstellung von Vitamin E bzw. Fettsäurepräparate üblichen sonstigen Hilfsstoffe enthalten.

Diese Mittel enthalten pro Dosiseinheit 50-1000 mg EPA und/oder DHA sowie 40-1000 mg Vitamin E.

In der folgenden Tabelle sind Versuchsdaten über die Abhängigkeit der Quickzeit von der Applikation von ungesättigten Fettsäuren und der Gabe von Vitamin E zusammengestellt.

| I | II | III | IV |
|---|---|---|---|
| Anzahl der Probanden n=112 | Kontrollwerte vor Omega-3-Fettsäuretherapie | Kontrollwerte 6 Wochen nach Omega-3-Fettsäure-therapie (bis 1.000 mg EPA/DHA) | Kontrollwerte 10 Wochen nach Omega-3-Fettsäuren-therapie und 5 Wochen Vitamin-E-Therapie (Omega-3-Fettsäuren + 500 mg Vitamin E) |
| Prothrombinzeit (Quick-Wert) in % der Norm = 100 % | 96 % n=112 | 58 % n=112 | 95 % n=112 |
| Vitamin E (Tocopherol) Norm: 0,5-1,6mg/100 ml | persönlicher "Normwert" = 100% n=112 | 60 % vom "Normwert" 100% n=112 | 100 % - Rückkehr im Normbereich n=112 |

Erläuterung:

Anzahl der Probanden n=112
Prothrombinwert = Quick in Sek. = 12 Sek. = 100 %
Vitamin-E = Normwert Erwachsene 0,5 - 1,6 mg/100 ml

Die in den Kästen angegebenen Prozentwerte sind bei 112 Probanden gemittelte-Werte in %. Die Veränderungen in den Blöcken III + IV sind gemittelte Werte in %, bei Prothrombinzeit als Absolut-Werte der Quick-Bestimmung, bei Vitamin-E in Prozent vom Ausgangswert, als Mittelwert.

**Patentansprüche**

**1.** Verwendung von Vitamin E zur Herstellung von Arzneimitteln zur Normalisierung der Blutgerinnung bei der Applikation von hochungesättigten Omega-3-Fettsäuren, wie EPA und DHA, **dadurch gekennzeichnet,** daß 40-100 Gew.-% Vitamin E, bezogen auf die Menge der hochungesättigten Fettsäuren, verwendet wird.

**2.** Verwendung gemäß Anspruch 1, **dadurch gekennzeichnet,** daß die Arzneimittel das Vitamin E und die ungesättigten Fettsäuren nebeneinander enthalten.

**3.** Arzneimittel oder Diätetika enthaltend ungesättigte Fettsäuren, wie EPA und DHA sowie Vitamin E, **dadurch gekennzeichnet,** daß der Gehalt an Vitamin E 40-100 Gew.-% der ungesättigten Fettsäuren beträgt und pro Dosiseinheit 50-1000 mg EPA und/oder DHA sowie 40-1000 mg Vitamin E enthalten sind.

**Claims**

**1.** The use of vitamine E for the preparation of medicaments for use in normalizing the blood coagulation while applying omega-3-type highly unsaturated fatty acids, like EPA and DHA, characterized in using 40 to 100 weight percent vitamine E in relation to the amount of highly unsaturated fatty acids.

**2.** The use according to claim 1, characterized in a medicament containing vitamine E and the unsaturated fatty acids side by side.

**3.** Medicaments or dietetics comprising highly unsaturated fatty acids, like EPA and DHA as well as vitamine E characterized in containing 40-100 weight percent vitamine E in relation to the unsaturated fatty acids and per dosis unit 50-1000 mg EPA and/or DHA as well as 40-100 mg vitamine E.

**Revendications**

**1.** Utilisation de vitamine E pour l'obtention des médicaments destinés à normaliser la coagulation sanguine lors de l'application d'acides gras oméga-3, tels que EPA et DHA caractérisée par le fait que 40-100 % en poids de vitamine E, relatifs à la quantite d'acides gras hautement insaturés, sont utilisés.

**2.** Utilisation suivant la revendication 1, caractérisée par le fait que les médicaments contiennent parallèlement la vitamine E et les acides gras insaturés.

**3.** Des médicaments ou produits diététiques contiennent des acides gras, tels que EPA et DHA ainsi que la vitamine E, caractérisés par le fait que la teneur en vitamine E représente 40-100 % en poids des acides gras insaturés et que 50-1000 mg de EPA et/ou DHA ainsi que 40-1000 mg de vitamine E sont contenus.